# FASCICULE DE BREVET EUROPEEN

(11) **EP 0 493 264 B1**
(45) Date de publication et mention de la délivrance du brevet: **04.09.1996**
(21) Numéro de dépôt: 91403555.5
(22) Date de dépôt: 27.12.1991
(51) Int. Cl.: C12N 1/20, C12P 19/06, C12R 1/64

(54) **Souche mutante de Xanthomonas campestris, procédé d'obtention de xanthane et xanthane non visqueux**
Mutanter Stamm von Xanthomonas Campestris, Verfahren zur Herstellung von Xanthan und nichtviskoser Xanthan
A mutant strain of Xanthomonas campestris, methods of producing xanthan and non-viscous xanthan

(30) Priorité: 28.12.1990 FR 9016515
(43) Date de publication de la demande: 01.07.1992
(73) Titulaire: SYSTEMS BIO-INDUSTRIES, F-92100 Boulogne (FR); ELF AQUITAINE, F-92400 Courbevoie (FR)
(72) Inventeur: Salome, Marc, F-31320 Castanet-Tolosan (FR)
(74) Mandataire: Gillard, Marie-Louise

(56) Documents cités:
- EP-A- 0 112 661
- US-A- 4 377 637

## Description

La présente invention a pour objet une souche mutante de Xanthomonas campestris, un procédé de fermentation de ce microorganisme pour l'obtention de xanthane, un xanthane non visqueux susceptible d'être produit par ledit procédé.

La viscosité des solutions aqueuses de xanthane a conduit à utiliser ce polysaccharide comme épaississant et stabilisant de suspensions dans les industries alimentaires, cosmétiques et pharmaceutiques, dans les peintures et les encres ainsi que pour la récupération assistée du pétrole.

Le xanthane est un polymère dont la chaîne principale, qui a la même structure que celle de la cellulose, porte des motifs latéraux trisaccharidiques substitués par des grouoes acétates ou pyruvates. On sait que selon les souches productrices et les conditions de culture, le polysaccharide produit peut avoir des motifs latéraux plus ou moins modifiés et une masse moléculaire moyenne variable, quoique toujours très élevée. Ceci peut entraîner des variations de ses propriétés physiques, notamment de sa viscosité ; néanmoins, il n'a jamais été isolé de polysaccharide ayant la structure du xanthane et dont la viscosité des solutions aqueuses soit pratiquement nulle.

On a certes décrit, dans le brevet US-A-4,377,637, un procédé pour la production de xanthane à viscosité réduite. Ladite réduction de viscosité, qui reste d'un pourcentage relativement faible, est obtenue grace à l'intervention d'anions sulfate.

On a maintenant trouvé qu'une souche de Xanthomonas campestris donnait dans de bonnes conditions de productivité, un xanthane de très faible viscosité.

Selon un premier asoect, l'invention concerne la souche de Xanthomonas campestris déposée, selon le traité de Budapest, à la Collection Nationale de Culture de Microorganismes (CNCM) France le 15 Juin 1990, sous le No. I-956.

Cette souche mutante a été sélectionnée sur un milieu liquide contenant de l'azote minéral comme seule source d'azote ainsi que du glycérol comme seule source de carbone. Elle peut être cultivée en milieu liquide synthétique ainsi que sur milieu solide.

Le microorganisme se présente sous forme de bâtonnets, et ses colonies jaunes et muqueuses, sur milieu solide, en présence de glucose, ne se distinguent pas de celles de la souche d'origine NRRL B-1459.

La souche mutante se distingue par sa croissance rapide sur glycérol comme seule source de carbone, par comparaison à la souche d'origine NRRL B-1459 et à la souche ATCC 31600 qui est capable de croître sur milieu synthétique ; elle est résistante à la streptomycine et à l'acide nalidixique et n'assimile pas le lactose.

Selon un autre de ses aspects, l'invention concerne un procédé de fermentation de ce microorganisme pour la production de xanthane classique ou non visqueux.

Ce procédé d'obtention de xanthane comprend une phase de production qui doit se dérouler en présence de calcium et d'hexamétaphosphate alcalin, de préférence de sodium.

Le milieu destiné à la phase de croissance de la souche mutante - phase de multiplication du microorganisme - comporte de préférence du glycérol comme seule source de carbone ; les autres composants dudit milieu sont ceux habituellement rencontrés dans les fermentations de Xanthomonas, que l'homme du métier choisira, à l'aide de quelques essais préalables, pour obtenir une croissance optimale. Ainsi à une source d'azote qui peut être minérale, comme un sel d'ammonium, ou plus complexe, on ajoutera des sels fournissant le phosphate, le magnésium, le potassium, le soufre, ainsi que le fer, le calcium et des oligo-éléments.

On pourra aussi mettre dans le milieu, un mélange tampon qui fixera le pH vers 7, par exemple à base d'acide 3-morpholinopropanesulfonique.

Le glycérol est assimilé rapidement par la souche mutante, y compris en présence d'un mélange d'acides aminés. En l'absence d'autre source de carbone, la production inutile et gênante de xanthane est supprimée, au cours de cette première phase.

Une source d'azote convenable sera constituée d'un mélange d'hydrolysat de caséine, d'acide glutamique et d'extrait de levure. Par ailleurs, la présence de bore, parmi les oligo-éléments, favorise la croissance du microorganisme. Le diluant du milieu de croissance est de l'eau, désionisée pour qu'il n'y ait pas d'interférence avec les ions introduits.

Le milieu destiné à la phase de production du xanthane, classique ou non visqueux, par la souche de l'invention, comporte : une source de carbone classique qui conduirait, avec une souche ordinaire, à la production de xanthane visqueux, comme un sucre ; une ou plusieurs sources d'azote ; des sels fournissant les ions habituels dans les fermentations de ce type, tel que phosphate, magnésium, potassium, soufre ainsi que fer et calcium et une solution d'oligo-éléments.

En outre, et c'est une caractéristique de l'invention, le milieu de production contient un hexamétaphosphate alcalin, à une concentration initiale comprise entre 54 et 500 mg/l et de préférence entre 200 et 300 mg/l, ce qui nécessite la présence de 1 mg à 15 mg de Ca⁺⁺/l.

L'hexamétaphosphate alcalin est avantageusement de l'hexamétaphosphate de sodium.

Le diluant du milieu de production est de l'eau désionisée.

On préfère comme source de carbone, le fructose, et mieux le glucose ; et comme source d'azote, la lysine, seule ou en mélange avec un hydrolysat de caséine.

Pour la préparation, selon l'invenvion, d'un xanthane non visqueux, le milieu doit, pendant toute la fermentation, rester non visqueux, facile à agiter et à aérer, et la fermentation doit être arrêtée dès que la viscosité du milieu commence à croître ; en effet, si celle-ci est continuée, du xanthane de viscosité normale apparaît peu à peu dans le milieu, et on peut n'obtenir, aprés une fermentation prolongée, que du xanthane de viscosité normale. Toutefois, l'addition, dès que la viscosité commence à croître, d'hexamétaphosphate alcalin, à raison de 50 à 200 mg/l de milieu, et de préférence autour de 100 mg/l, permet de suspendre l'augmentation de la viscosité et de prolonger la production du xanthane non visqueux.

Ledit hexamétaphosphate intervenant ici est, aussi avantageusement, de l'hexamétaphosphate de sodium.

En général, la durée de la fermentation est en moyenne de 30 heures avant de constater l'apparition d'une viscosité dans le moût de fermentation, cependant, elle peut être continuée pendant 10 heures, et davantage, en faisant une addition d'hexamétaphosphate dans le milieu.

La fermentation peut être arrêtée à tout moment avant l'apparition de viscosité, mais on attend de préférence que la totalité de la source de carbone soit pratiquement consommée.

Le milieu en fin de fermentation, n'étant pas visqueux, la séparation des corps cellulaires et des insolubles éventuels est effectuée facilement, par centrifugation, par microfiltration ou par ultrafiltration ; dans ce dernier cas, les cellules peuvent être recyclées et la fermentation reprise, après simple addition, dans le milieu où elles sont restées en suspension, des constituants nécessaires du milieu de production.

Les solutions de polysaccharide ainsi obtenues sont limpides et peuvent, pour certaines applications, être utilisées telles que.

Le polysaccharide peut aussi être isolé, avant ou après séparation des insolubles, par précipitation selon une méthode connue : on ajoute dans le milieu un solvant miscible à l'eau dans lequel le polysaccharide est insoluble tel que l'acétone, l'éthanol ou de préférence l'isopropanol ; comme pour une fermentation classique, la présence de faibles concentrations d'ions alcalins ou alcalino-terreux, et notamment de calcium, dans le milieu, entre 50 et 100 mM, améliore la précipitation.

Pour certaines applications, par exemple en cosmétique, il peut être souhaitable d'isoler un xanthane non contaminé par des lipopolysaccharides, dont l'activité pyrogène est bien connue. Or, on sait que les Xanthomonas étant des bactéries Gram -, des quantités importantes de lipopolysaccharides sont libérées dans le milieu de fermentation ; elles pourront être éliminées par précipitation avant celle du polysaccharide par des techniques connues,telles que l'addition d'ions alcalinoterreux dans un milieu basique.

Pour la préparation, selon l'invention, de xanthane visqueux - classique -, on fait produire la souche de Xanthomonas de l'invention dans un milieu contenant un hexamétaphosphate alcalin à une concentration comprise entre 50 mg/l et 500 mg/l, de préférence entre 200 et 300 mg/l, et des ions calcium à raison de 1 à 15 mg de Ca⁺⁺/l, et on prolonge la fermentation jusqu'à ce que la viscosité du milieu soit comparable à celle d'un milieu de production usuel, avant d'isoler le xanthane selon les procédés classiques. Dans ce contexte de production de xanthane visqueux, on peut également prolonger la fermentation en milieu non visqueux en ajoutant, dès que la viscosité commence à croître, de l'hexamétaphosphate alcalin, à raison de 50 à 200 mg/l de milieu et de préférence autour de 100 mg/l. On diffère ainsi l'apparition dudit xanthane visqueux.

Un tel procédé est avantageux dans la mesure où le milieu de production est visqueux pendant une durée nettement plus courte que dans une fermentation classique, de même productivité. On diminue ainsi nettement la consommation d'énergie d'agitation.

Le milieu de production, pour l'obtention de xanthane visqueux ou non visqueux, présente les mêmes caractéristiques, à savoir, la présence d'un hexamétaphosphate alcalin - de préférence l'hexamétaphosphate de sodium - et d'ions calcium.

Selon un dernier aspect, l'invention concerne un nouveau xanthane, non visqueux. Ledit xanthane non visqueux est susceptible d'être obtenu par le procédé décrit ci-dessus, procédé qui fait intervenir de façon caractéristique la souche de Xanthomonas campestris CNCM I-956 dans un milieu renfermant un hexamétaphosphate alcalin et qui est interrompu opportunément.

Par xanthane non visqueux, on entend un produit dont les solutions aqueuses à la concentration de 10 g/l, contenant environ 1 % (p/v) de KCl, ont une viscosité inférieure à 700 mPa.s et mieux à 250 mPa.s, mesurée avec un viscosimètre Brookfield, avec un mobile LVT, équipé d'une aiguille 1 ou 2 tournant à 60 tours/minute à 25°C, alors que pour les xanthanes connus, elle est comprise entre 1200 et 1500 mPa.s · Cette mesure de viscosité est effectuée de façon classique en présence de KCl pour donner un résultat indépendant de la quantité d'ions qui peuvent être introduits dans l'eau avec le xanthane à étudier.

Ce xanthane non visqueux est de composition chimique identique aux xanthanes connus en particulier en ce qui concerne les groupes acétates et pyruvates ; par contre, la masse moléculaire moyenne du polymère est inférieure à celle des xanthanes visqueux.

Par une technique couplant la chromatographie d'exclusion et la diffusion de la lumière laser à faible angle, décrite par exemple dans Carbohydrate Polymers 6, p. 477-492 (1986), on a déterminé que les masses moléculaires des xanthanes du commerce étaient de 2 x 10⁶ ou plus, tandis que la masse moléculaire moyenne du polysaccharide selon l'invention est de l'ordre de 6 à 7 x 10⁵.

Les solutions de concentration 1 à 20 g/l du xanthane non visqueux, selon l'invention, ne deviennent pas visqueuses par chauffage, entre 80 et 120°C et pendant 5 à 30 minutes.

Un xanthane non visqueux présentant ces caractéristiques structurelles, n'a jamais été décrit, et l'invention n'est pas limitée au polysaccharide produit par la souche CNCM I-956 ; elle concerne aussi les polysaccharides non visqueux, ayant la structure chimique du xanthane et qui pourraient être produits par une autre souche mutante de Xanthomonas campestris ou par un microorganisme d'espèce ou de genre différent, éventuellement obtenu par génie génétique, ou encore qui serait préparé par synthèse chimique ou hémisynthèse.

Le xanthane non visqueux selon l'invention, peut être utilisé seul pour son pouvoir suspensoïde, mais pour les applications classiques du xanthane usuel, qui font appel à des propriétés viscosifiantes et épaississantes, il peut être mélangé en proportions variables à un xanthane visqueux usuel pour obtenir des compositions de xanthane de viscosités intermédiaires, de propriétés reproductibles et présentant néanmoins les caractéristiques rhéologiques intéressantes du xanthane, c'est-à-dire l'indépendance de la viscosité de ses solutions pour une large gamme de température et de pH et sa pseudoplasticité; ces mélanges auront les mêmes applications comme agent épaississant et suspensoïde que le xanthane classique, puisque le xanthane non visqueux conserve ses caractéristiques de solubilité.

Ces compositions de xanthanes, comme le xanthane non visqueux selon l'invention, pourront être associées aux autres hydrocolloïdes, tels que carraghénanes, pectines, gélatines, alginates et caroubes, dans leurs applications habituelles, notamment dans l'industrie alimentaire.

En outre, le xanthane non visqueux pourra être utilisé pour son pouvoir filmogène, notamment en boulangerie.

Enfin, le xanthane non visqueux, selon l'invention, pourra être soumis à des réactions chimiques plus facilement qu'un xanthane visqueux pour donner par greffage ou réticulation des produits utiles.

On décrit dans ce qui suit, des exemples de mise en oeuvre de l'invention.

### Exemple 1

Obtention du xanthane non visqueux en fermenteur de laboratoire.

La souche Xanthomonas campestris mutante CNCM I-956 peut être conservée de façon classique lyophilisée ou en tube congelé à - 80°C en milieu aqueux glycérolé à 20 %.

### a) Phase de croissance.

Le milieu de croissance est préparé préalablement :

On dissout dans 800 ml d'eau désionisée :

FeSO₄, 7H₂O(0,125 g) ; CaCl₂, 2H₂O(0,015 g) ; MgSO₄, 7H₂O(0,300 g) ; K₂SO₄(0,040 g) ; NaCl(0,125 g) ; acide glutamique (3,0 g) ; extrait de levure (1,0 g) ; hydrolysat de caséine (7,5 g), et on ajoute une solution d'oligo-éléments (1 ml).

L'hydrolysat de caséine est, par exemple, celui commercialisé par HUMKO SHEFFIELD (USA) sous le nom "Hy-Case, salt free" ; la solution d'oligo-éléments est préparée par dissolution dans 900 ml d'eau désionisée de : 10 g de MnCl₂, 2 g de ZnSO₄, 7H₂O, 25 mg de CuSO₄, 5H₂O, 2 g de CoCl₂, 6H₂O, 0,5 g de H₃BO₃; 180 mg de KI, 1,9 g de Na₂MoO₄, 2H₂O, 2,4 g d'AlCl₃, 6H₂O, 50 mg de KCr(SO₄)₂, 12H₂O et 26 mg de Ni SO₄, 6H₂O ; on ajoute 100 ml d'acide chlorhydrique concentré et on met à l'autoclave à 120°C pendant 30 minutes.

Le pH de la solution est amené à 7,4 par addition d'une solution aqueuse de KOH (d = 1,38) avant maintien à 120°C pendant 45 minutes. On ajoute ensuite 100 ml d'une solution aqueuse filtrée de glycérol (18,4 g) et de K₂HPO₄(1,5 g).

Une préculture des souches lyophilisées est effectuée en fiole, agitée à 30°C, dans ce milieu, puis 150 ml de préculture sont introduits dans 1,35 l de milieu dans un fermenteur maintenu entre 28°C et 32°C ; le pH est maintenu à 7,4 par addition de NaOH ou HCl, et le milieu est aéré par introduction d'air frais sous une légère pression.

On poursuit la culture jusqu'à obtention d'environ 8 g de biomasse par litre.

### b) Phase de production.

Le milieu de production est constitué d'une solution dans 800 ml d'eau désionisée d'hydrolysat de caséine (1,13 g) ; MgSO₄, 7H₂O(50 mg); MgCl₂, 6H₂O(85 mg); CaCl₂, 2H₂O(27 mg); lysine (1 g), dont le pH a été amenè à 7 par addition de KOH avant stérilisation ; mélangée à une solution dans 100 ml d'eau désionisée de K₂HPO₄(4 g) d'hexamétaphosphate de sodium (250 mg) et de glucose (45 g).

On introduit 150 ml du moût obtenu en fin de phase de croissance dans 1,35 l de milieu de production ; durant la fermentation, la température est maintenue entre 28°C et 32°C, le pH à 7 ; la concentration en glucose a une valeur supérieure à 10 g/l, et le milieu est aéré par introduction d'air frais, sous légère pression.

### c) Isolement du xanthane non visqueux.

On mélange, à 80°C, 4 g du moût de fermentation avec 30 ml d'eau, puis on ajoute 90 ml d'isopropanol ; après repos du mélange, on filtre et sèche le solide à 55°C pendant 15 heures.

Dans le Tableau I, on a porté le poids de xanthane ainsi isolé par kilo de milieu, ainsi que la viscosité du milieu de fermentation dont il est extrait.

Les mesures de viscosité ont été effectuées à 25°C avec un viscosimètre Brookfield, équipé d'un mobile LVT, avec une aiguille 2 ou 3 tournant à 30 tours/minute.

**TABLEAU I**

| **durée de la production** | **viscosité du moût (mPa.s)** | **Poids de xanthane (g/kg)** |
|---|---|---|
| 21 h 30 | 22 | 10,6 |
| 24 h | 32 | 11,8 |
| 26 h 30 | 52 | 14,25 |
| 29 h | 87 | 14,8 |
| 31 h | 130 | 15,7 |
| 46 h | 5400 | 26,3 |
| 50 h | 7100 | 27,1 |

### d) Séparation des cellules.

2 g de solide obtenu selon c) sont mis en suspension dans 100 ml d'eau, et le mélange est centrifugé à 12000 g pendant 30 minutes. Le culot de centrifugation est séparé, et on précipite le xanthane par addition d'isopropanol dans la solution limpide.

### Exemple 2

Dans une autre fermentation, aprés 24 heures de production, la viscosité du moût est de 10 mPa.s, et le poids du xanthane de 13,75 g/kg de moût ; on ajoute alors 100 mg/l d'hexamétaphosphate de sodium dans le moût. Dans ces conditions, après 40 heures, la viscosité est de 20 mPa.s et le poids de xanthane de 20,40 g/kg.

## Revendications

1. Souche de Xanthomonas campestris déposée à la CNCM sous le No. I-956.

2. Procédé d'obtention de xanthane caractérisé en ce que l'on cultive la souche de Xanthomonas campestris CNCM I-956 dans un milieu nutritit contenant 50 à 500 mg/l d'un hexamétaphosphate alcalin et 1 à 15 mg/l d'ions calcium.

3. Procédé selon la revendication 2, caractérisé en ce que la phase de croissance du microorganisme a lieu dans un milieu contenant du glycérol comme seule source de carbone.

4. Procédé selon l'une des revendications 2 ou 3, caractérisé en ce qu'on utilise de l'hexamétaphosphate de sodium.

5. Procédé selon l'une quelconque des revendications 2 à 4, pour l'obtention d'un xanthane dont la viscosité des solutions aqueuses à 10 g/l, à 25°C, est inférieure à 700 mPa.s, caractérisé en ce que la fermentation est arrêtée lorsque la viscosité du milieu augmente.

6. Procédé selon l'une quelconque des revendications 2 à 5, caractérisé en ce qu'on introduit dans le moût de fermentation de 50 à 200 mg d'hexamétaphosphate alcalin par litre de milieu lorsque la viscosité du milieu de fermentation s'accroit.

7. Xanthane susceptible d'être obtenu par le procédé selon l'une des revendications 5 ou 6, dont la viscosité des solutions aqueuses à 10g/l, à 25°C, est inférieure à 700 mPa.s.

8. Xanthane susceptible d'être obtenu par le procédé selon l'une des revendications 5 ou 6, dont la viscosité des solutions aqueuses à 10g/l, à 25°C, est inférieure à 250 mPa.s.

## Patentansprüche

1. Xanthomonas campestris-Stamm, hinterlegt bei der CNCM unter der Nr. I-956.

2. Verfahren zum Erhalten von Xanthan, dadurch gekennzeichnet, daß der Xanthomonas campestris-Stamm CNCM I-956 in einem Nährstoffmedium, das 50 bis 500 mg/l eines alkalischen Hexametaphosphats und 1 bis 15 mg/l Calciumionen enthält, kultiviert wird.

3. Verfahren nach Anspruch 2, dadurch gekennzeichnet, daß die Wachstumsphase des Mikroorganismus in einem Medium, das Glycerin als einzige Kohlenstoffquelle enthält, stattfindet.

4. Verfahren nach Anspruch 2 oder 3, dadurch gekennzeichnet, daß Natriumhexametaphosphat verwendet wird.

5. Verfahren nach einem der Ansprüche 2 bis 4 zum Erhalten eines Xanthans, dessen Viskosität wässeriger Lösungen bei 10 g/l, bei 25°C, weniger als 700 mPa.s beträgt, dadurch gekennzeichnet, daß die Fermentation angehalten wird, wenn die Viskosität des Mediums zunimmt.

6. Verfahren nach einem der Ansprüche 2 bis 5, dadurch gekennzeichnet, daß in die Fermentationsflüssigkeit 50 bis 200 mg alkalisches Hexametaphosphat pro 1 Medium eingebracht werden, wenn die Viskosität des Fermentationsmediums steigt.

7. Xanthan, welches durch das Verfahren nach Anspruch 5 oder 6 erhältlich ist, dessen Viskosität wässeriger Lösungen bei 10 g/l, bei 25°C, weniger als 700 mPa.s beträgt.

8. Xanthan, welches durch das Verfahren nach Anspruch 5 oder 6 erhältlich ist, dessen Viskosität wässeriger Lösungen bei 10 g/l, bei 25°C, weniger als 250 mPa.s beträgt.

## Claims

1. The strain of Xanthomonas campestris deposited in the CNCM under n° I-956.

2. Process for obtaining xanthan, characterized in that Xanthomonas campestris CNCM 1-956 is cultivated in a nutrient medium containing 50 to 500 mg/l of an alkali metal hexametaphosphate and 1 to 15 mg/l of calcium ions.

3. Process according to claim 2, characterized in that the growth phase of the microorganism takes place in a medium containing glycerol as the only carbon source.

4. Process according to one of claims 2 or 3, characterized in that sodium hexametaphosphate is used.

5. Process according to any one of claims 2 to 4, for obtaining a xanthan whose aqueous solutions at a concentration of 10 g/l have a viscosity of less than 700 mPa.s at 25°C, characterized in that fermentation is stopped when the viscosity of the medium increases.

6. Process according to any one of claims 2 to 5, characterized in that 50 to 200 mg of an alkali metal hexametaphosphate per liter of medium are introduced into the fermentation broth when the viscosity of the fermentation medium increases.

7. Xanthan capable of being obtained by the process according to one of claims 5 or 6 whose aqueous solutions at a concentration of 10 g/l have a viscosity of less than 700 mPa.s at 25°C.

8. Xanthan capable of being obtained by the process according to one of claims 5 or 6 whose aqueous solutions at a concentration of 10 g/l have a viscosity of less than 250 mPa.s at 25°C.
